# EUROPEAN PATENT APPLICATION

(11) **EP 2 042 207 A1**
(43) Date of publication of application: **01.04.2009**
(21) Application number: 06781191.9
(22) Date of filing: 18.07.2006
(51) Int. Cl.: A61M 11/00

(54) **PORTABLE DRUG SOLUTION CONTAINER**

(71) Applicant: Satsumaya Syouten Co.,Ltd., Setagaya-ku Tokyo 154-0011 (JP)
(72) Inventor: KUROKI, Kiyomi, Setagaya-ku Tokyo 154-0011 (JP)
(74) Representative: Siebert, Karsten
(86) International application number: PCT/JP2006/314178
(87) International publication number: WO 2008/010262

(57) **Abstract**

[PROBLEMS] To provide a portable drug solution container that is filled with nearly one dose of throat drug solution, ensuring easy unsealing and use and realizing unsealing with the direction of nozzle fixed. [MEANS FOR SOLVING PROBLEMS] There is provided disposable portable drug solution container (1) filled with a small amount of drug solution, including platelike member (5) capable of being folded at given position into at least two segments and, bonded fast to the backside of the platelike member (5), bag member (2) filled with a drug solution, and further including nozzle (6) extending from given position of the container (1), wherein in the state of having the platelike member (5) folded, there is realized communication of the nozzle (6) with the interior of the bag member (2).

## Description

### TECHNICAL FIELD

The present invention relates to a disposable portable drug solution container filled with a throat drug solution that corresponds substantially to a single dose.

### BACKGROUND ART

Conventionally, there are throat sprayers in which a mouthwash medication or other drug solution used in the treatment of throat inflammation or the like is stored in a container for a liquid drug, and these sprayers are provided with a downwardly-pressed spray head in which a long vaporizing nozzle is provided to the upper part of the drug solution container (e.g., see Patent Document 1).

Patent Document 1: Japanese Laid-Open Patent Application No. 2002-355307 (Page 2 and FIG. 1).

### DISCLOSURE OF THE INVENTION

### [Problems to Be Solved By the Invention]

The throat sprayer disclosed in Patent Document 1 can be stored in a bag or the like and carried, and is designed to store a large amount of drug solution in the drug solution container so that the drug solution can be vaporized a plurality of times. Consequently, the overall shape of the throat sprayer is large and is not suited to be a container that is readily portable by storing the throat sprayer in a pocket or the like.

The present invention was contrived in view of such problems, and an object of the present invention is to provide a disposable portable drug solution container filled with a throat drug solution that corresponds substantially to a single dose, the portable drug solution container being capable of being readily opened and used, and being capable of being opened in a state in which the direction of the nozzle is fixed.

### [Means Used to Solve the Above-Mentioned Problems]

In order to solve the above-described problems, the portable drug solution container according to a first aspect of the present invention is a disposable, portable drug solution container filled with a small amount of a drug solution, the portable drug solution container characterized in that a sack unit filled with a drug solution is fastened on a rear surface of a plate-shaped unit that can be bent in at least two divisions at a predetermined position; a nozzle extends from a predetermined location of the container; and the nozzle and the interior of the sack unit are made to be in communication in a state in which the plate-shaped unit is bent.
According to this aspect, not only can a portable drug solution container be realized that is easily carried in a compact shape in which the drug solution is filled, e.g., with a single dose, but the container can also readily be used through bending the plate-shaped unit into two divisions. Moreover, when the plate-shaped unit has begun to bend, the drug solution in the sack unit is sprayed from the nozzle. This is done after the user has aimed the nozzle in a state in which the plate-shaped unit is bent without the drug solution filled in the sack unit being sprayed. Sudden scattering of the solution can be prevented when the portable drug solution container is opened.

The portable drug solution container according to a second aspect of the present invention is the portable drug solution container according to the first aspect, and is characterized in that the nozzle and the interior of the sack unit are made to be in communication by using a pressing force when the plate-shaped unit is made to bend.
According to this aspect, the drug solution inside the sack unit is sprayed from the nozzle by using the pressing force when the plate-shaped unit is bent, and the opening action of the portable drug solution container is facilitated.

The portable drug solution container according to a third aspect of the present invention is the portable drug solution container according to the first or second aspect, and is
characterized in that the plate-shaped unit forms a flat-plate shape and is bendable on the rear surface; a concave part in which a protruding part is disposed inside is formed in the rear surface, the nozzle is in communication with the concave part, the protruding part is caused to puncture the sack unit in the concave part by a pressing force generated when the plate-shaped unit, and the drug solution inside the sack unit flows into the concave part and is sprayed from the nozzle.
According to this aspect, the drug solution is sprayed in a direction of a fixed target by the nozzle because the nozzle is in communication with the concave part, and the sack unit is reliably ruptured by the protruding part inside the concave part.

The portable drug solution container according to a fourth aspect of the present invention is the portable drug solution container according to the third aspect, and is characterized in that the sack unit is held between the bent plate-shaped unit, the pressure of the drug solution inside the sack unit increases, and the sack unit expands toward the concave part, whereby the protruding part punctures the sack unit.
According to this aspect, the drug solution spraying action is facilitated because the sack unit is brought close to the protruding part by using the increase in the fluid pressure inside the sack unit brought about by bending the plate-shaped unit, and the sack unit can be punctured by the protruding part.

The portable drug solution container according to a fifth aspect of the present invention is the portable drug solution container according to any of first to fourth aspects, and is characterized in that the nozzle extending from one of the plate-shaped units separates from the other of the plate-shaped units during bending into two divisions.
According to this aspect, the nozzle will not become an encumbrance, and the nozzle can be prevented from breaking while being carried, because the portable drug solution container can be carried in a state wherein the nozzle is accommodated in the plate-shaped unit before being bent. Also, the direction of the drug solution spray can easily be established because the nozzle protrudes from the plate-shaped unit after the plate-shaped unit is bent.

The portable drug solution container according to a sixth aspect of the present invention is the portable drug solution container according any of the first to fifth aspects, and is characterized in that the plate-shaped unit has a substantially discoid shape.
According to this aspect, the plate-shaped unit can be prevented from catching onto the edges of a pocket when the user inserts the portable drug solution container into a pocket or the like, or pulls the portable drug solution container from a pocket or the like, because corners are not formed on the plate-shaped unit.

### BRIEF DESCRIPTION OF THE DRAWINGS

FIG. 1 is an exploded perspective view showing the mouthwash container according to example 1;
FIG. 2(a) is a perspective view showing the mouthwash container prior to being opened, (b) is a perspective view of the mouthwash container when being opened, and (c) is a perspective view showing the mouthwash container after being opened;
FIG. 3(a) is a cross-sectional view showing the mouthwash container prior to being opened, (b) is a cross-sectional view of the mouthwash container when being opened, and (c) is a cross-sectional view showing the mouthwash container after being opened;
FIG. 4 is a view showing an example of using the mouthwash container;
FIG. 5 is a perspective view showing a storage case for storing a plurality of mouthwash containers;
FIG. 6(a) is a perspective view showing a mouthwash container prior to being opened, according to example 2, (b) is a perspective view of the mouthwash container when being opened, and (c) is a perspective view showing the mouthwash container after being opened;
FIG. 7(a) is a cross-sectional view showing the mouthwash container prior to being opened, (b) is a cross-sectional view of the mouthwash container when being opened, and (c) is a cross-sectional view showing the mouthwash container after being opened;
FIG. 8(a) is a perspective view showing a mouthwash container prior to being opened, according to example 3, (b) is a perspective view of the mouthwash container when being opened, and (c) is a perspective view showing the mouthwash container after being opened;
FIG. 9 (a) is a cross-sectional view showing the mouthwash container prior to being opened, (b) is a cross-sectional view of the mouthwash container when being opened, and (c) is a cross-sectional view showing the mouthwash container after being opened; and
FIG. 10 is a perspective view showing a state in which a plurality of mouthwash containers is connected.

### Key

1, 20, 32 Mouthwash containers (portable drug solution containers)
2, 21, 34 Sack units
3 First plate member
4 Second plate member
23 Plate member
5, 24, 33 Plate-shaped units
6, 25, 37 Nozzle parts
7, 26 Fitted parts (sealing means)
8, 27, 40 Concave parts
9, 28, 39 Distal end aperture parts
10, 29, 41 Protruding parts
11, 30 Membrane parts (sack units)
42 Membrane unit (sack unit)
12 Engagement piece (sealing means)
31, 38 Seal units (sealing means)

### BEST MODE FOR CARRYING OUT THE INVENTION

A best mode for carrying out the portable drug solution container according to the present invention will be described below on the basis of examples.

### Example 1

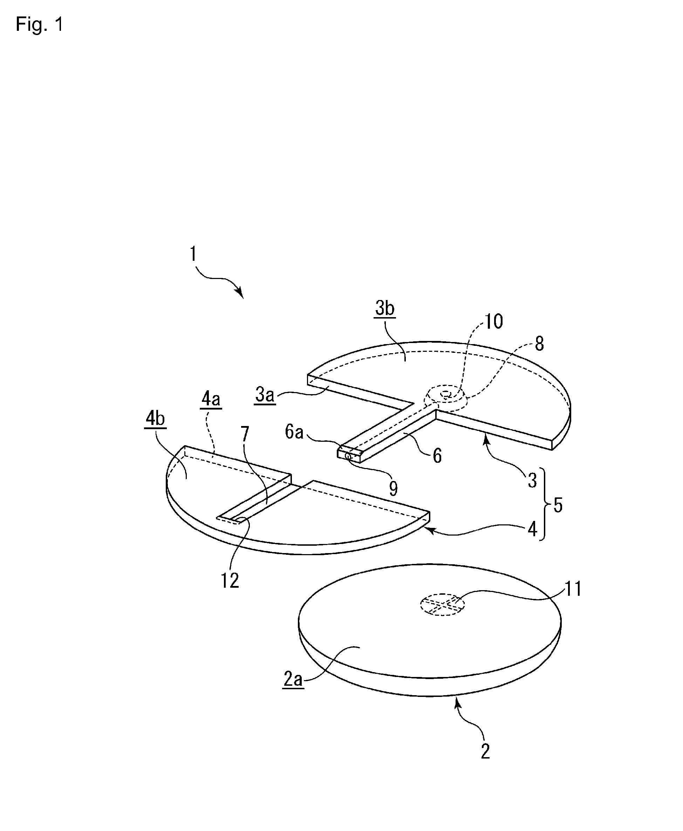

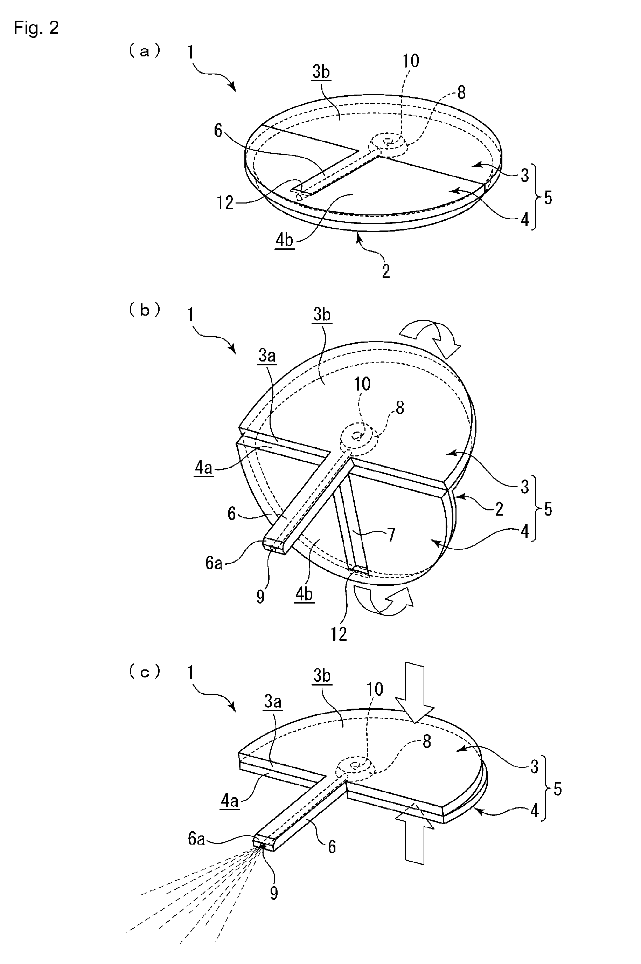

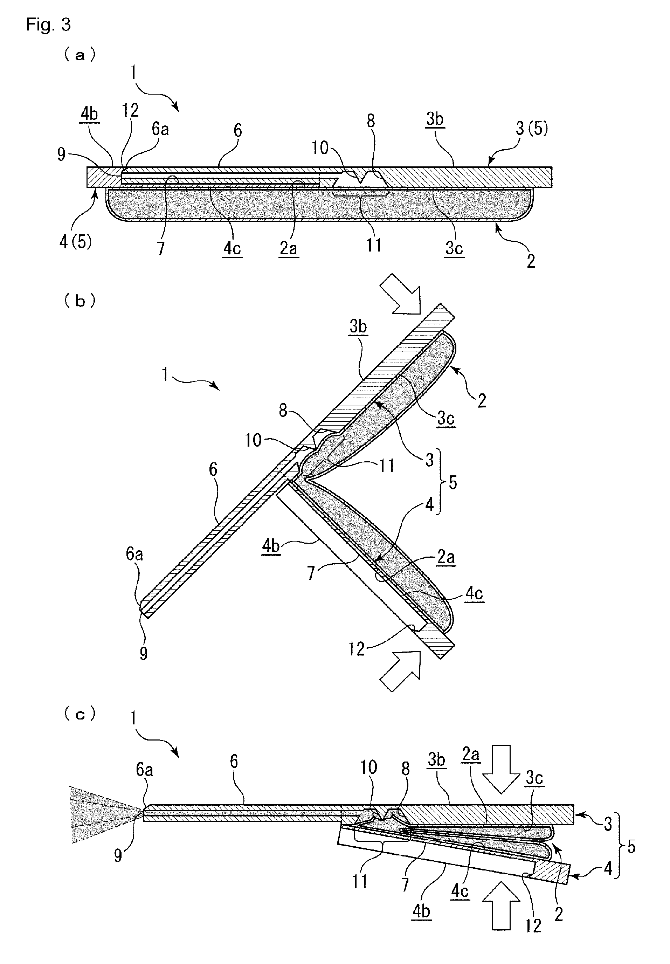

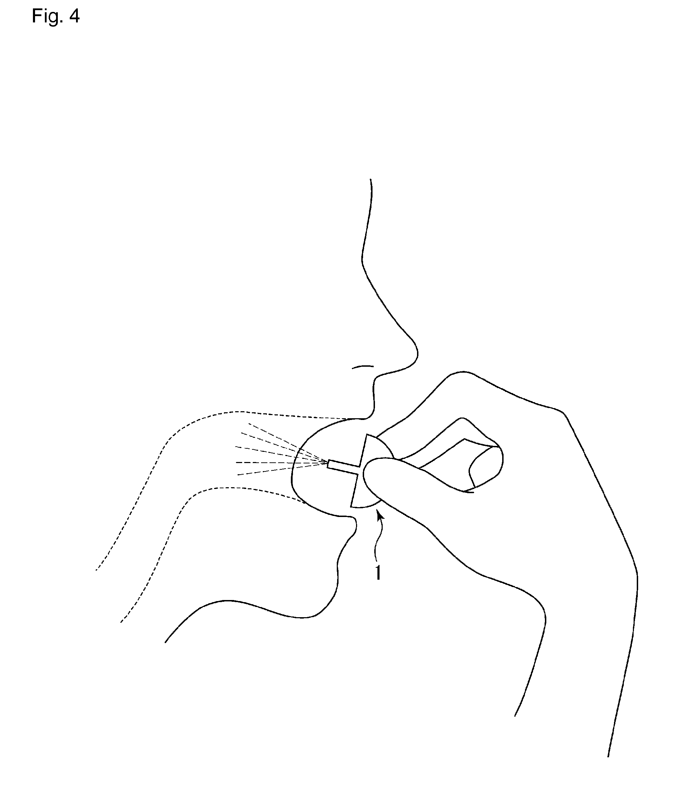

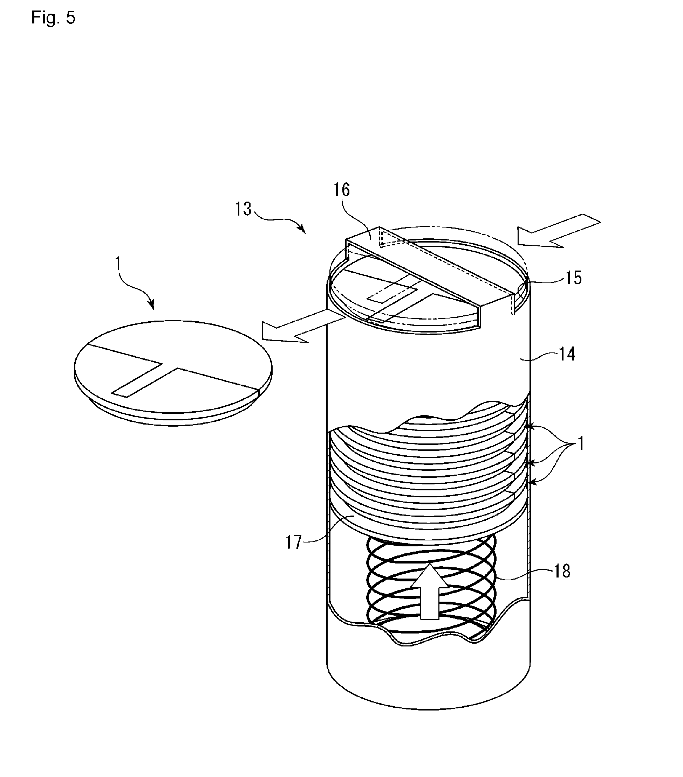

An example of the present invention is described on the basis of drawings, wherein, first, FIG. 1 is an exploded perspective view showing a mouthwash container according to the example 1; FIG. 2(a) is a perspective view showing the mouthwash container prior to being opened, FIG. 2(b) is a perspective view of the mouthwash container when being opened, and FIG. 2(c) is a perspective view showing the mouthwash container after being opened; FIG. 3(a) is a cross-sectional view showing the mouthwash container prior to being opened, FIG. 3 (b) is a cross-sectional view of the mouthwash container when being opened, and FIG. 3(c) is a cross-sectional view showing the mouthwash container after being opened; FIG. 4 is a view showing an example of using the mouthwash container; and FIG. 5 is a perspective view showing a storage case for storing a plurality of mouthwash containers.

Reference numeral 1 of FIG. 1 is the mouthwash container as the disposable, portable drug solution container according to example 1 designed so as to be portable after being filled with a mouthwash medication or other drug solution for use in treating throat inflammation or the like. The mouthwash container 1 comprises a substantially discoid-shaped sack unit 2 filled with a liquid mouthwash medication corresponding to a single dose, and a first plate member 3 and a second plate member 4 that are connected to each other, each having a substantially semicircular shape.

The first plate member 3 and the second plate member 4 are connected together by using an adhesive or the like on the two corresponding contact surfaces 3a and 4a, as shown in FIG. 1. When the first plate member 3 and the second plate member 4 are connected, a plate-shaped unit 5 having a discoid shape is formed (see FIG. 2(a)). The first plate member 3 and the second plate member 4 are bonded at a sufficient strength such that when a user pinches the peripheral end parts of the plate-shaped unit 5 using his fingers and applies force, the plate-shaped unit 5 bends and the contact surfaces 3a and 4a of the first plate member 3 and the second plate member 4 separate from each other.

A long nozzle part 6 extends from the first plate member 3, and a long groove-shaped fitted part 7 into which the nozzle part 6 of the first plate member 3 is fitted is formed on the surface 4b of the second plate member 4. Also, the length of the nozzle part 6 is formed to be about half the diameter of the plate-shaped unit 5 having a discoid shape, and the nozzle part 6 can be accommodated within the dimensions of the plate-shaped unit 5 (see FIG. 2).

A concave part 8 is formed on the rear surface 3c of the first plate member 3, and the concave part 8 is in communication with the distal end aperture part 9 of the nozzle part 6, as shown in FIGS. 2 and 3(a). Also, a substantially conically shaped protruding part 10 that protrudes toward the opening of the concave part 8 is formed inside the concave part 8. The distal end of the protruding part 10 is formed so as not to protrude from the rear surface 3c of the first plate member 3, and so that the protruding length of the protruding part 10 is less than the depth of the concave part 8.

Formed on the sack unit 2 is a fastening surface 2a that is fastened to the rear surfaces 3c, 4c of the plate-shaped unit 5 in which the first plate member 3 and the second plate member 4 are joined to one another, and a cross-shaped half cut membrane part 11 is formed in the fastening surface 2a in a location that corresponds to the concave part 8 in the rear surface 3c of the first plate member 3, as shown in FIG. 1.

The sack unit 2 is configured so that the entire fastening surface 2a other than the membrane part 11 is fastened to the entirety of the rear surfaces 3c, 4c of the plate-shaped unit 5 using an adhesive agent or the like. Also, when the sack unit 2 is fastened to the plate-shaped unit 5, the membrane part 11 that is formed on the fastening surface 2a of the sack unit 2 is arranged so as to cover the aperture of the concave part 8, and the internal part of the sack unit 2 and the interior part of the concave part 8 are partitioned by membrane part 11, as shown in FIGS. 2(a) and 3(a).

A notch part 6a that is notched on the external surface 3b side of the first plate member 3 is formed on the distal end of the nozzle part 6 of the first plate member 3, and an engagement piece 12 for engaging the notch part 6a of the nozzle part 6 is formed on the second plate member 4, as shown in FIG. 3(a). The notch part 6a of the nozzle part 6 is engaged by the engagement piece 12 of the fitted part 7, and is thereby configured so that the nozzle part 6 does not disengage from the fitted part 7 when an external force is unexpectedly applied to the plate-shaped unit 5. The fitted part 7 and the engagement piece 12 constitute sealing means in the example 1.

Next, an example of the use of the mouthwash container 1 will be described. The mouthwash container 1 prior to being opened is substantially discoid in shape, as shown by FIG. 2(a). For this reason, the mouthwash container 1 can be stored in a bag, a pocket of the user, or the like and easily carried. Particularly since corners are not formed on the plate-shaped unit 5 comprising the first plate member 3 and the second plate member 4, the plate-shaped unit 5 can be prevented from catching onto the edges of a pocket when the user inserts the mouthwash container 1 into a pocket or the like, or pulls the mouthwash container 1 from a pocket or the like. Also, the mouthwash container 1 is filled with a single dose of the mouthwash medication, and is small and readily portable.

The user pinches the peripheral end parts of the plate-shaped unit 5 when the mouthwash container 1 is to be used, whereby the plate-shaped unit 5 is bent toward the rear surfaces 3c, 4c so as to form a letter V shape, as shown in FIGS. 2(b) and 3(b). At this point, the contact surfaces 3a, 4a of the first plate member 3 and the second plate member 4 that constitute the plate-shaped unit 5 separate from each other, the nozzle part 6 is disengaged from the fitted part 7, and the nozzle part 6 protrudes from the plate-shaped unit 5.

When the nozzle part 6 is in a state disengaged from the fitted part 7, i.e., when the plate-shaped unit 5 has started to bend, the user can aim the nozzle part 6 toward his own throat because the mouthwash medication inside the sack unit 2 has still not been sprayed (see FIG. 4). In the state in which the contact surfaces 3a and 4a are separated from each other, the first plate member 3 and the second plate member 4 are connected via the sack unit 2.

The sack unit 2 is pinched between the first plate member 3 and the second plate member 4, and the sack unit 2 is compressed, as shown in FIGS. 2(c) and 3(c). Further, the user pinches the first plate member 3 and the second plate member 4 between his fingers and applies a pressing force so that the two rear surfaces 3c, 4c approach one another, whereupon the fluid pressure of the mouthwash medication inside the sack unit 2 increases and the membrane part 11 of the sack unit 2 expands toward the concave part 8. In other words, the membrane part 11 enters inside the concave part 8.

The expanded membrane part 11 makes contact with the distal end of the protruding part 10 disposed inside the concave part 8, and the protruding part 10 punctures the membrane part 11. In this arrangement, a cross-shaped half cut (see FIG. 1) is formed in the membrane part 11, whereby the protruding part 10 more readily punctures the membrane part 11.

The mouthwash medication inside the sack unit 2 flows from the hole opened in the membrane part 11 into the concave part 8 by the puncturing action of the protruding part 10. The mouthwash medication that has flowed into the concave part 8 passes through the nozzle part 6 that is in communication with the concave part 8 and is sprayed from the distal end aperture part 9 of the nozzle part 6. Also, the mouthwash medication is sprayed vigorously from the distal end aperture part 9 of the nozzle part 6 because a predetermined liquid pressure is applied inside the compressed sack unit 2 (see FIG. 4).

The sack unit 2 that is pinched by the two rear surfaces 3c, 4c of the first plate member 3 and the second plate member 4 is configured so that the volumetric capacity of the interior of the sack unit can be compressed to zero volume. Accordingly, the user can cause all the mouthwash medication inside the sack unit 2 to flow out from the interior of the sack unit 2 by merely pinching with his fingers.

The long nozzle part 6 is provided to the first plate member 3, whereby the distal end aperture part 9 of the nozzle part 6 can be brought close to the throat by inserting the nozzle part 6 inside the mouth, and the mouthwash medication can be sprayed accurately toward the throat, as shown by FIG. 4.

Also, the nozzle part 6 is accommodated within the dimensions of the plate-shaped unit 5 prior to being bent, and is formed to a length that protrudes from the plate-shaped unit 5 (the first plate member 3) after being bent, whereby the nozzle part 6 can be extended in order to more easily establish the direction of the spray of the mouthwash medication. Also, the nozzle part 6 will not become an obstruction, and breakage of the nozzle part 6 can be prevented when the container is carried around because the mouthwash container 1 can be carried in a state in which the nozzle part 6 is accommodated in the plate-shaped unit 5.

The mouthwash container 1 according to the present example is designed to be discarded after use, but when mouthwash container 1 is used and there is no garbage receptacle or the like available near the user, the used mouthwash container 1 must be stored again in a pocket, a bag, or the like of the user. In such a case, the contact surfaces 3a and 4a of the first plate member 3 and the second plate member 4 can be placed in contact with one another again, the shape of the plate-shaped unit 5 is returned to the discoid-shaped state prior to use (see FIG. 2 (a)), whereupon the nozzle part 6 is fitted again in the fitted part 7.

At this point, the notch part 6a that is formed at the distal end of the nozzle part 6, which is fitted in the fitted part 7, is engaged by the engagement piece 12 provided to the fitted part 7; the nozzle part 6 is prevented from disengaging from the fitted part 7; and the distal end aperture part 9 of the nozzle part 6 is sealed.

In this way, the engagement piece 12 and the fitted part 7 are provided as sealing means capable of closing the distal end aperture part 9 of nozzle part 6 at least after the mouthwash container 1 (sack unit 2) has been opened, whereby the mouthwash medication that has remained in the concave part 8 and the nozzle part 6 can be prevented from seeping out from the distal end aperture part 9 of the nozzle part 6 after the mouthwash container 1 has been opened, and the inside of the pocket or the like is not stained by leakage when the used mouthwash container 1 is again placed in a pocket or the like. Since the mouthwash container 1 is formed in a discoid shape even after use, the mouthwash container 1 can be placed in a pocket or the like and is readily portable.

The mouthwash container 1 of the present example is configured so that the plate-shaped unit 5 having a discoid shape (flat-plate shape) can be bent in the direction of the rear surfaces 3c, 4c, the concave part 8 inside which the protruding part 10 is disposed is formed on the rear surfaces 3c, 4c of the plate-shaped unit 5, and the sack unit 2 filled with the mouthwash medication (liquid) is attached. The mouthwash medication inside the sack unit 2 is sealed by the membrane part 11 that partitions the interior part of the sack unit 2 and the interior part of the concave part 8. The nozzle part 6 for spraying mouthwash medication is in communication with the concave part 8, the protruding part 10 punctures the membrane part 11 due to a pressing force when the user bends the plate-shaped unit 5, and the mouthwash medication in the sack unit 2 flows into the concave part 8 and is sprayed from the nozzle part 6, whereby the mouthwash medication in the sack unit 2 is not sprayed from nozzle part 6 in a state in which the user begins to bend the plate-shaped unit 5 by the action of pinching with his fingers. However, after the user determines the direction of the nozzle part 6, the protruding part 10 punctures and opens the membrane part 11 by further applying a pressing force and bending the plate-shaped unit 5, the mouthwash medication inside the sack unit 2 is sprayed from the nozzle part 6, and sudden scattering of the mouthwash medication can be prevented when the mouthwash container 1 is opened.

Also, the configuration in one in which the sack unit 2 is held between the bent plate-shaped unit 5 (first plate member 3 and second plate member 4), the pressure of the mouthwash medication in the sack unit 2 increases, the membrane part 11 expands toward the concave part 8, and the protruding part 10 punctures the membrane part 11, whereby the user can puncture the membrane part 11 with the aid of the protruding part 10 by merely bending the plate-shaped unit 5 to increase the liquid pressure of the mouthwash medication inside the sack unit 2 and to make the membrane part 11 approach the protruding part 10 using the liquid pressure, and the action of opening and spraying the mouthwash medication can be facilitated.

Also, the mouthwash container 1 is readily portable not only when carrying mouthwash medication on a trip or a business trip, but also when going out and the like, because the mouthwash medication can readily be carried in an amount corresponding to a single dose. Also, the user can select a suitable number of units of the portable mouthwash container 1 and carry the mouthwash medication for the number of times he will spray the mouthwash medication. Furthermore, the mouthwash medication can be preserved over a long period without deterioration or decay because the mouthwash medication is sealed inside the sack unit 2 prior to being used.

When a plurality of mouthwash containers 1 is carried, a storage case 13 can be used for storage as shown in FIG. 5. The storage case 13 has a case unit 14 having a cylindrical shape that can store a plurality of mouthwash containers 1 in a stacked state. An opening part 15 is formed on the upper end of the case unit 14, and a restraining slat 16 is formed so as to traverse the opening part 15. Also, a pressing plate 17 for partitioning the interior part of the case unit 14 is provided inside the case unit 14, and the pressing plate 17 is pressed and urged toward the opening part 15 by a coil spring 18 provided below the pressing plate 17.

The plurality of mouthwash containers 1 is pressed toward the opening part 15 via the pressing plate 17 by the urging force of the coil spring 18, and the mouthwash container 1 disposed at the opening part 15 is restrained by the restraining slat 16 and does not fly out to the exterior of the case unit 14. The restraining slat 16 is provided at a position projecting from the upper end part of the case unit 14 and is designed so that a single mouthwash container 1 is exposed to the exterior from the opening part 15.

When the mouthwash container 1 is removed from the storage case 13, the user presses with a finger a side of a mouthwash container 1 restrained by the restraining slat 16 and exposed from the opening part 15. At this point, the mouthwash container 1 is slidably moved to the side and released from the restraining slat 16, allowing the storage case 13 to be removed. When this type of storage case 13 is used, a plurality of mouthwash containers 1 can be carried without being cumbersome.

### Example 2

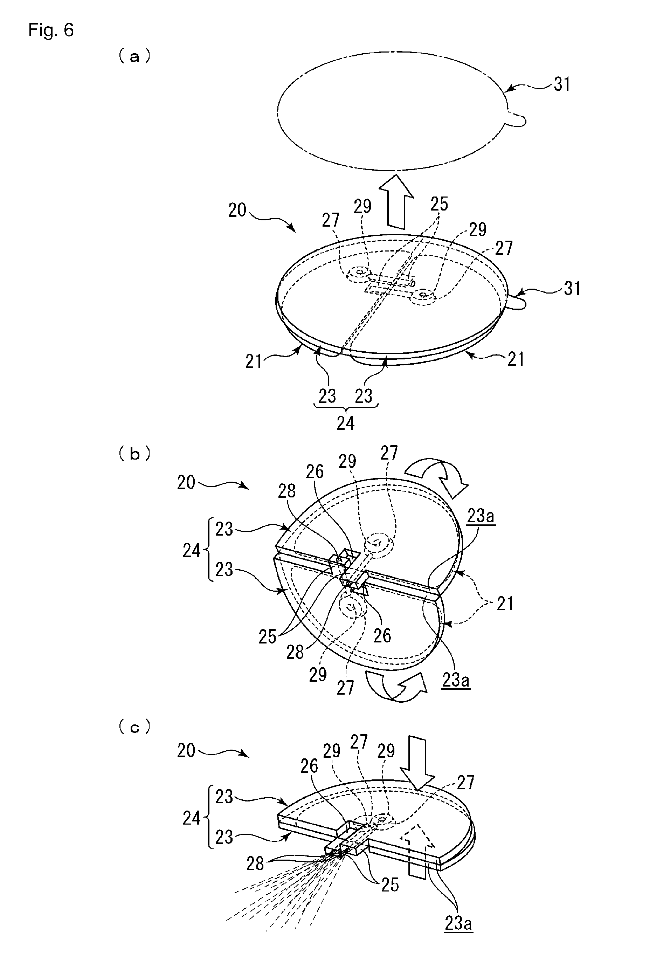

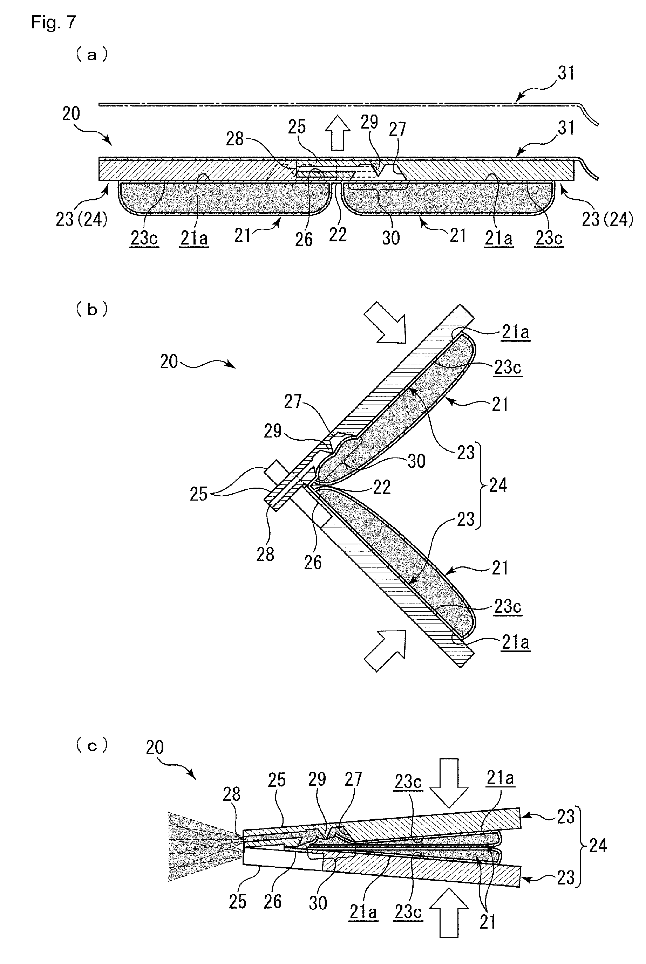

Next, a mouthwash container 20 according to example 2 will be described with reference to FIGS. 6 and 7. FIG. 6(a) is a perspective view showing a mouthwash container 20 prior to being opened in example 2; FIG. 6(b) is a perspective view of the mouthwash container 20 when being opened; and FIG. 6(c) is a perspective view showing the mouthwash container 20 after being opened. FIG. 7(a) is a cross-sectional view showing the mouthwash container 20 prior to being opened; FIG. 7(b) is a cross-sectional view of the mouthwash container 20 when being opened; and FIG. 7(c) is a cross-sectional view showing the mouthwash container 20 after being opened.

The mouthwash container 20 as the portable drug solution container according to example 2 is filled with the liquid mouthwash medication corresponding to a single dose divided and filled in two sack units 21, the two sack units 21 are connected to each other by a connecting membrane 22 extending from the sack units 21, as shown in FIGS. 6(a) and 7(a). Also, [each sack unit] has a substantially semicircular shape and has two plate members 23 having the same shape. The contact surfaces 23a of each of the plate members 23 are bonded to one another, and a plate-shaped unit 24 having a discoid shape is formed.

A short nozzle part 25, and a short groove-shaped fitted part 26 into which the corresponding nozzle part 25 is fitted, are formed on each plate member 23. Concave parts 27 are formed on the rear surface 23c of each plate member 23, the concave parts 27 are in communication with the distal end aperture part 28 of the nozzle part 25, and a protruding part 29 is formed inside the concave parts 27.

The plate members 23 are joined to one another, and a fastening surface 21a of the sack units 21 filled with mouthwash medication is fastened on the rear surface 23c of each plate member 23, as shown in FIGS. 6(a) and 7(a). Membrane parts 30 (sack units) are formed in which a cross shape half cut is formed in the same manner as in example 1 in the fastening surface 21a of each sack unit 21. The membrane part 30 that is formed on the fastening surface 21a of each sack unit 21 is arranged so as to cover the aperture of each concave part 27 when the sack units 21 are fastened to the plate members 23, and the interior part of the sack unit 21 and the interior part of the concave part 27 are partitioned by the membrane part 30.

A seal unit 31 for covering the entire surface 23a of the plate-shaped unit 24 is applied to the surface 23a of the discoid plate-shaped unit 24 formed by joining the two plate members 23. The plate-shaped unit 24 does bend, and the nozzle part 25 will not become disengaged from the fitted part 26, when an external force is unexpectedly applied to the plate-shaped unit 24 because the seal unit 31 has been applied. The fitted part 26 and the seal unit 31 constitute sealing means in example 2.

The user pinches the peripheral end parts of the plate-shaped unit 24 when the mouthwash container 20 is to be used, whereby the plate-shaped unit 24 is bent toward the rear surface 23c so as to form a V shape, as shown in FIGS. 6(b) and 7(b). At this point, the two contact surfaces 23a of the plate member 23 separate, and the nozzle parts 25 disengage from the fitted parts 26 and protrude from the plate-shaped unit 24.

When the plate-shaped unit 24 has started to bend, the user can aim the nozzle part 25 and set a target toward his own throat because the mouthwash medication inside the sack unit 21 has not yet been sprayed. In a state when the two contact surfaces 23a are separated, the plate members 23 are connected via the connecting membrane 22 for connecting the two sack units 21.

The two sack units 21 are held between the two plate members 23 and compressed, as shown in FIGS. 6(c) and 7(c). Further, when the user pinches the two plate members 23 with his fingers and applies a pressing force in the direction that brings the two rear surfaces 23c close one another, fluid pressure of the mouthwash medication inside the sack units 21 increases and the membrane parts 30 of the sack units 21 expand toward the concave parts 27. The expanded membrane part 30 contacts the distal end of the protruding part 29 disposed in the concave part 27, and the protruding part 29 punctures the membrane part 30.

The mouthwash medication in the sack unit 21 flows into concave part 27 from the hole opened in the membrane part 30 by the puncturing action of the protruding part 29, passes through the nozzle part 25 that is in communication with the concave part 27, and is sprayed from the distal end aperture part 28 of the nozzle part 25.

After the mouthwash container 20 has been used, the contact surfaces 23a of the plate members 23 are again caused to make contact, and the nozzle part 25 is again fitted in the fitted part 26 when the shape of the plate-shaped unit 24 returns to the discoid shape prior to use (see FIG. 6(a)). The seal unit 31 is then applied to the surface 23a of the plate-shaped unit 24. The mouthwash medication remaining in the concave part 27 and the nozzle part 25 can be prevented from seeping out of the distal end aperture part 28 of the nozzle part 25 because the distal end aperture part 28 of the nozzle part 25 is sealed by the fitted part 26 and the seal unit 31.

### Example 3

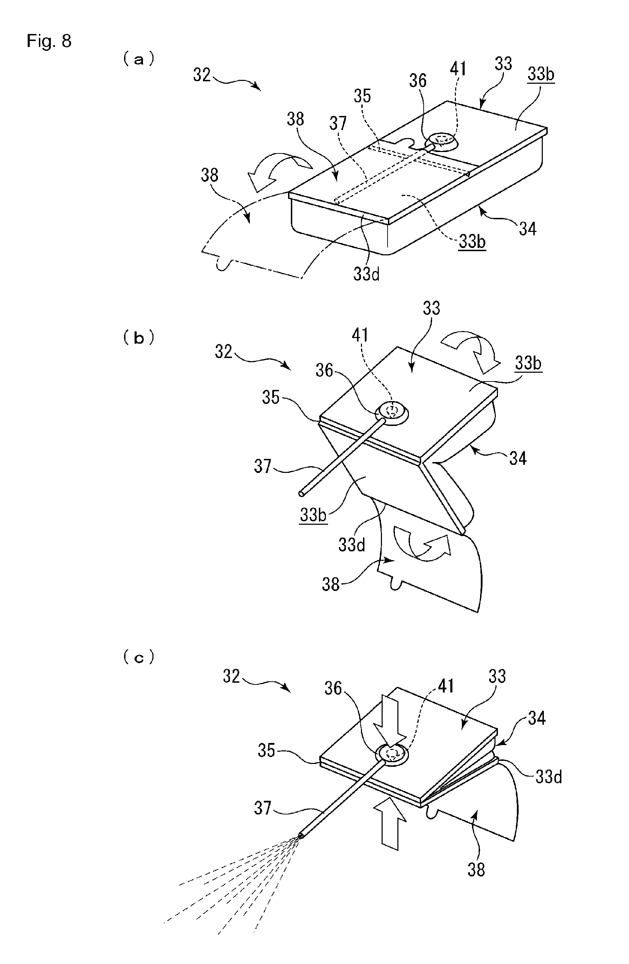

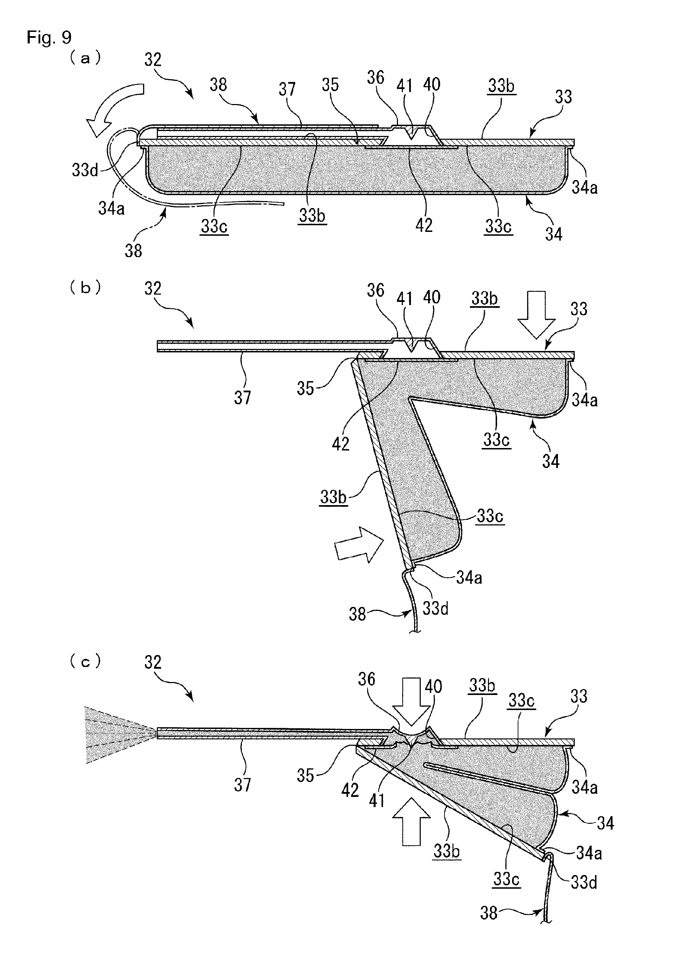

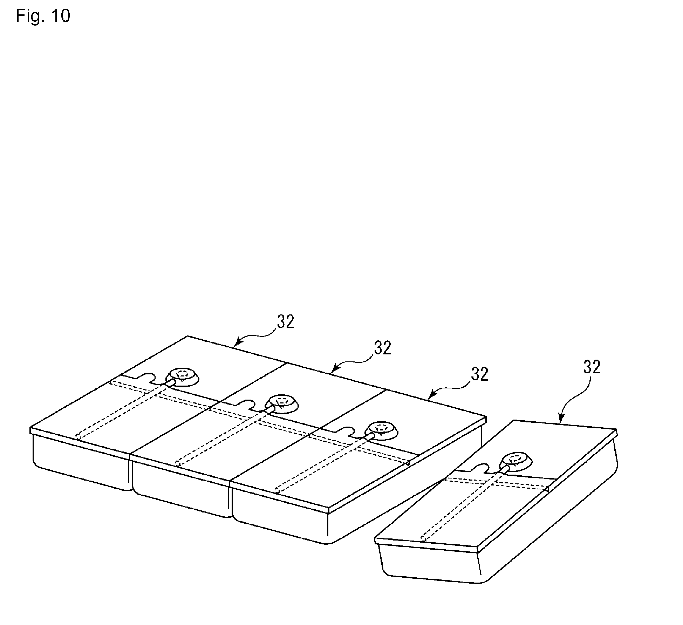

Next, a mouthwash container 32 according to an example 3 will be described with reference to FIGS. 8 and 9. FIG. 8(a) is a perspective view showing a mouthwash container 32 prior to being opened in example 3; FIG. 8(b) is a perspective view of the mouthwash container 32 when being opened; and FIG. 8(c) is a perspective view showing the mouthwash container 32 after being opened. FIG. 9(a) is a cross-sectional view showing the mouthwash container 32 prior to being opened; FIG. 9(b) is a cross-sectional view of the mouthwash container 32 when being opened; and FIG. 9(c) is a cross-sectional view showing the mouthwash container 32 after being opened. FIG. 10 is a perspective view showing the state in which a plurality of mouthwash containers 32 is connected.

The mouthwash container 32 as the portable drug solution container according to example 3 has a plate-shaped unit 33 that forms a rectangular shape (flat-plate shape), and a sack unit 34 filled with liquid mouthwash medication corresponding to a single dose, as shown in FIGS. 8(a) and 9(a). A half-cut part 35 is formed in the center of the surface 33b of the plate-shaped unit 33. When a user pinches the end parts of the plate-shaped unit 33 with his fingers and applies a force, the plate-shaped unit 33 is made to bend at the half-cut part 35.

A pressing part 36 formed so as to expand outward is provided on the surface 33b of the plate-shaped unit 33, and a long nozzle part 37 extending along the surface 33b of the plate-shaped unit 33 is provided on the pressing part 36. The nozzle part 37 extends so as to cross over the half-cut part 35, and the distal end of the nozzle part 37 extends to the edge part 33d of the plate-shaped unit 33.

Also, a sealing unit 38 as sealing means in example 3 is mounted on the edge part 33d of the plate-shaped unit 33. When the sealing unit 38 is applied to the surface 33b of the plate-shaped unit 33, the distal end aperture part 39 of the nozzle part 37 is covered by the sealing unit 38. Also, the sealing unit 38 extends so as to cross over the half-cut part 35 of the plate-shaped unit 33. The sealing unit 38 is applied to the plate-shaped unit 33, and the plate-shaped unit 33 is prevented from bending at the half-cut part 35 when an external force is unexpectedly applied to the plate-shaped unit 33.

A concave part 40 is formed on the rear surface of the pressing part 36 provided to the plate-shaped unit 33, the concave part 40 is in communication with the distal end aperture part 39 of the nozzle part 37, and a protruding part 41 is formed in the interior part of the concave part 40. A membrane unit 42 (sack unit) in the form of a sheet covering the concave part 40 is applied to the rear surface 33c of the plate-shaped unit 33. The membrane unit 42 in example 3 is a unit that is separate from the sack unit 34.

The entire peripheral edge part 34a of the sack unit 34 in which the liquid mouthwash medication is held is welded onto the rear surface 33c of the plate-shaped unit 33 with the aid of a heat seal, and the mouthwash medication is sealed by the plate-shaped unit 33 and the sack unit 34 and filled. The interior part of the sack unit 34 and the interior part of the concave part 40 are partitioned by the membrane unit 42.

The user pinches the two end parts of the plate-shaped unit 33 when the mouthwash container 32 is to be used, and the plate-shaped unit 33 is bent toward the rear surface 33c so that the plate-shaped unit 33 forms a V shape, as shown in FIGS. 8(b) and 9(b). At this point, the plate-shaped unit 33 bends at the half-cut part 35, and the nozzle part 37 protrudes from the plate-shaped unit 33. When the plate-shaped unit 33 has started to bend, the user can aim the nozzle part 37 and set a target toward his own throat because the mouthwash medication inside the sack unit 34 has yet to be sprayed.

The sack unit 34 is held and compressed between parts of the bent plate-shaped unit 33, as shown in FIGS. 8(c) and 9(c). Further, the user pinches the plate-shaped unit 33 with his fingers and presses the pressing part 36 with his fingers, the protruding part 41 inside the concave part 40 protrudes from the concave part 40, and the distal end of the protruding part 41 punctures the membrane unit 42.

The configuration is one in which the pressing part 36 provided to the surface 33b of the plate-shaped unit 33 is pressed and the protruding part 41 protrudes from inside the concave part 40 and punctures the membrane unit 42, whereby the pressing force generated when the pressing part 36 is pressed is transmitted immediately to the protruding part 41 and the puncturing of the membrane unit 42 by the protruding part 41 is facilitated.

The mouthwash medication inside the sack unit 34 flows from the hole opened in the membrane unit 42 into the concave part 40 by the puncturing action of the protruding part 41, passes through the nozzle part 37 that is in communication with the concave part 40, and is sprayed from the distal end aperture part 39 of the nozzle part 37.

After the mouthwash container 32 has been used, the shape of the plate-shaped unit 33 is returned to the state of the flat-plate shape prior to being used (see FIG. 8(a)), and the sealing unit 38 is applied to the surface 33b of the plate-shaped unit 33. At this point, the mouthwash medication that has remained in the concave part 40 and the nozzle part 37 can be prevented from seeping out from the distal end aperture part 39 of the nozzle part 37 because the distal end aperture part 39 of the nozzle part 37 is sealed by the sealing unit 38.

A plurality of the mouthwash containers 32 may be manufactured in advance for use in a state in which each of the edge parts of the plate-shaped units 33 is joined so that a single mouthwash container 32 is separated and used during application, as shown in FIG. 10. In this way, the mouthwash container 32 can be prevented from scattering when being carried and stored in a pocket, bag or the like, by connecting a plurality of such mouthwash containers 32.

Examples of the present invention have been described above with reference to the drawings, but a concrete configuration is not limited to the above-described examples, and modifications and additions may be included in the present invention in a range that does not depart from the spirit of the present invention.

For example, in the above-described examples, a liquid mouthwash medication is filled in the mouthwash container 1, 20, or 32 as the portable drug solution container according to the present invention, but the portable drug solution container according to the present invention may be used as a container for filling eye medications and other drug solutions other than mouthwash medications, or ketchup, sauce or other liquid seasonings.

Also, the above-described examples have configurations wherein the nozzle parts 6, 25 protrude from contact surfaces 3a, 23a on the plate members 3, 23, and the mouthwash medication is sprayed from the distal end aperture parts 9, 28 of the nozzle parts 6, 25. However, the nozzle of the present invention in not limited to nozzle parts 3a, 23a that protrude from the plate members 3 and 23, and the configuration may be one in which an aperture part in communication with the concave part is provided to the contact surface of the plate members, whereby the aperture part can be configured as a nozzle to spray the mouthwash medication.

Also, in the above-described examples 1 and 2, the membrane parts 11, 30 are configured to be punctured by contact with the protruding parts 10, 29 by expanding into the concave parts 8, 27. However, the present invention is not limited to this, and rather than providing protruding parts 10, 29 inside the concave parts 8, 27, the mouthwash medication may be sprayed by, e.g., making the membrane parts 11, 30 burst due to an increase in liquid pressure of the mouthwash medication inside the sack units 2, 21 by forming the membrane parts 11, 30 more thinly than the other locations of the sack units 2, 21.

## Claims

1. A disposable, portable drug solution container filled with a small amount of a drug solution, the portable drug solution container **characterized in that:**
a sack unit filled with a drug solution is fastened on a rear surface of a plate-shaped unit that can be bent in at least two divisions at a predetermined position;
a nozzle extends from a predetermined location of said container; and
said nozzle and the interior of said sack unit are made to be in communication in a state in which said plate-shaped unit is bent.

2. The portable drug solution container according to claim 1, **characterized in that** said nozzle and the interior of said sack unit are made to be in communication by using a pressing force when said plate-shaped unit is made to bend.

3. The portable drug solution container according to claim 1 or 2, **characterized in that:**
said plate-shaped unit forms a flat-plate shape and is bendable on the rear surface;
a concave part in which a protruding part is disposed inside is formed in the rear surface;
said nozzle is in communication with said concave part;
said protruding part is caused to puncture the sack unit in said concave part by a pressing force generated when said plate-shaped unit is bent; and
the drug solution inside said sack unit flows into said concave part and is sprayed from said nozzle.

4. The portable drug solution container according to claim 3, **characterized in that** said sack unit is held between said bent plate-shaped unit, the pressure of the drug solution inside the sack unit increases, and said sack unit expands toward said concave part, whereby said protruding part punctures said sack unit.

5. The portable drug solution container according to any of claims 1 through 4, **characterized in that** said nozzle extending from one of the plate-shaped units separates from the other of the plate-shaped units during bending into two divisions.

6. The portable drug solution container according to any of claims 1 through 5, **characterized in that** said plate-shaped unit has a substantially discoid shape.
